# EUROPÄISCHE PATENTSCHRIFT

(11) **EP 2 415 396 B1**
(45) Veröffentlichungstag und Bekanntmachung des Hinweises auf die Patenterteilung: **17.08.2016**
(21) Anmeldenummer: 11173732.6
(22) Anmeldetag: 13.07.2011
(51) Int. Cl.: A61B 5/0215, A61B 5/04, A61N 1/362, A61N 1/365, A61N 1/368, A61N 1/37, A61N 1/39

(54) **Herzmonitor**
Heart monitor
Moniteur cardiaque

(30) Priorität: 06.08.2010 US 371175 P
(43) Veröffentlichungstag der Anmeldung: 08.02.2012
(73) Patentinhaber: BIOTRONIK SE & Co. KG, 12359 Berlin (DE)
(72) Erfinder: Kirchner, Jens, 91052 Erlangen (DE); Lippert, Michael, 91522 Ansbach (DE)
(74) Vertreter: Galander, Marcus

(56) Entgegenhaltungen:
- US-A1- 2003 074 029
- US-A1- 2004 172 079
- US-A1- 2005 027 323
- US-A1- 2005 049 646
- US-A1- 2007 043 299
- US-A1- 2007 191 724
- US-A1- 2008 097 226
- US-A1- 2010 113 945
- US-B1- 6 494 832

## Beschreibung

Die Erfindung betrifft einen Herzmonitor zur automatischen Erkennung von Zuständen möglicher Herzinsuffizienz.

Ein grundsätzlich bekannter Indikator für die Herzleistung ist z.B. die Kontraktilität des rechten Ventrikels.

Die Kontraktilität beschreibt die Kraft und die Geschwindigkeit einer myokardialen Kontraktion. Die Kontraktilität wird durch drei Mechanismen gesteuert:
eine direkte Steuerung durch das autonome Nervensystem (ANS),
den sogenannten Frank-Starling-Mechanismus, und
den sogenannten Bowditch-Effekt (Kraft-Frequenzkopplung).

Der Hauptmechanismus, die Steuerung der Kreislaufregulation durch das autonome Nervensystem, vergrößert die Kontraktilität und die Herzrate, wenn ein erhöhter metabolischer Bedarf vorliegt, beispielsweise bei körperlicher oder psychischer Anstrengung, um eine geeignete Blutversorgung sicherzustellen.

Bei Patienten mit chronischem Herzversagen (Chronic Heart Failure, HF) nimmt die myokardiale Kontraktilität bis auf ein niedriges Niveau ab und die interventrikuläre Synchronisation wird verschlechtert. Dies wird von einem geringen Auswurfanteil (Ejection Fraction, EF) begleitet, sowie von einer geringen Lebensqualität und einer hohen Sterblichkeit. HF kommt in der Bevölkerung häufig vor. HF-Patienten werden mit verschiedenen Medikamenten behandelt, die den inotropen Zustand beeinflussen, beispielsweise Betablockern, um die Herzrate zu stabilisieren, aber auch mit positiv inotropen Medikamenten, zum Beispiel Glykosiden, um die Kontraktilität zu erhöhen. In jüngerer Zeit werden HF-Patienten mit Resynchronisationstherapie-Geräten, beispielsweise 3-Kammer-Herzschrittmachern oder Defibrillatoren behandelt. Ziel einer solchen Schrittmachertherapie ist es, die zwei Ventrikel eines Herzens durch biventrikuläre Stimulation zu synchronisieren, um das Zeitverhalten der Herzkammern zu verbessern und damit die Herzleistung (Cardiac Resynchronisation Therapy, CRT).

Die Kontraktilität ist daher eine wichtige zu beobachtende Größe, insbesondere für HF-Patienten. Solche Beobachtung ist wichtig, um
den Zustand des Patienten und eine Linderung oder ein Fortschreiten der Krankheit zu beobachten,
eine Resynchronisationstherapie des Herzens (Cardiac Resynchronisation Therapy, CRT) festzulegen und zu beobachten, und
eine Medikamentenbehandlung zu beobachten.

Die Information über die Kontraktilität kann außerdem dazu verwendet werden, eine Herzschrittmachertherapie oder eine Therapie durch einen implantierbaren Kardioverter/Defibrillator (implantable Cardioverter/Defibrillator, ICD) zu optimieren.

Obwohl die Kontraktilität eine Größe von großer Wichtigkeit ist, ist sie in der klinischen Praxis schwer zu messen. Es ist bekannt, die Kontraktilität anhand eines maximalen ventrikulären Druckgradienten (dP/dt)ₘₐₓ im rechten Ventrikel oder im linken Ventrikel zu bestimmen. Ein linksventrikulärer Auswurfanteil kann außerdem mittels einer Echokardiographie bestimmt werden. Eine Untersuchung des rechten Ventrikels mittels Echokardiographie ist aus anatomischen Gründen sehr schwierig, obwohl die Information über den rechten Ventrikel für eine vollständige Untersuchung sehr wichtig ist. Beide Vorgehensweisen, die Druckmessung und die Echokardiographie sind zeitaufwendig und teuer. Die ventrikuläre Druckmessung erfordert einen invasiven Eingriff. Sie erfordert einen Druckkatheter in einem oder beiden Ventrikel und kann nur während einer elektrophysiologischen Studie oder der Implantation eines Herzschrittmachers oder Kardioverters/Defibrillators durchgeführt werden.

Implantate, mit denen die Kontraktilität eines Herzens ermittelt werden kann, sind beispielsweise in den US Patenten 4,674,518, US 5,417,717 und US 7,519,422 beschrieben. Dort erfolgt eine Messung der Änderung des Herzkammervolumens mittels des Druckgradientens dP/dt und mittels Impedanz-Plethysmografie.

Die der Erfindung zugrunde liegende Aufgabe ist es, eine verbesserte Vorrichtung zum Erkennen einer Herzinsuffizienz zu schaffen.

Erfindungsgemäß wird hierzu ein medizinisches Gerät nach Anspruch 1 vorgeschlagen.

Das erfindungsgemäße medizinische Gerät wertet somit die jeweils von den Mitteln zum Bestimmen der Kontraktilität des jeweiligen Ventrikels erfassten Kontraktilitätswerte in Abhängigkeit von den von den Mitteln zum Bestimmen der Vorlast des jeweiligen Ventrikels erfassten Vorlastenwerten aus und berücksichtigt dabei die Zuordnung der Vorlastwerte zu den Kontraktilitätswerten.

Aus US 2007/043299 A1 ist die Bestimmung der Kontraktiliät über den Herzton S1 oder alternativ über eine linksventrikuläre Druckmesseung sowie die Korrektur des so ermittelten Kontraktilitätswertes mit der Vorlast bekannt. US 2005/049646 A1 beschreibt die Bestimmung der Kontraktilität mittels kardialer Impedanzmessung. US 2007/191724 A1 und US 6 494 832 B1 sind weitere Beispiele für die Bestimmung der Kontraktilität mittels Druckmessung.

Die Erfindung schließt die Erkenntnis ein, dass bekannte Methoden, die ein gemitteltes (dP/dt)ₘₐₓ bestimmen, dabei die Unterschiede in den zugrunde liegenden Vorlasten vernachlässigen. Die funktionale Abhängigkeit von Kontraktilität und Vorlast wird somit von den bekannten Verfahren und Vorrichtungen nicht bestimmt.

Erfindungsgemäß wird vorgeschlagen, aus den respiratorischen Schwankungen von (dP/dt)ₘₐₓ ein Maß für den Frank-Starling-Mechanismus abzuleiten. Dieses dient zur Erkennung und Überwachung von Rechts- bzw. Linksherzinsuffizienz. Die Erfindung erlaubt ein Erkennen der Herzinsuffizienz durch Quantifizieren des Frank-Starling Effekts

Die Erfindung schließt außerdem die Erkenntnis ein, dass (dP/dt)ₘₐₓ ein an sich bekanntes Maß für die Kontraktilität des jeweils untersuchten Ventrikels bildet und mit den respiratorisch bedingten Druckschwankungen im Thorax und Abdomen ein Mechanismus zur Verfügung steht, über den spontan, d.h. nicht von extern vorgegeben, die Vorlast variiert wird. Die Stärke dieser Variation lässt sich dabei an respiratorischen Druckschwankungen (z.B. von Maximal- oder enddiastolischem Druck) ablesen.

Somit kann allein aus einer Blutdruckmessung der Frank-Starling-Mechanismus studiert, d.h. die Abhängigkeit der Kontraktilität von der Vorlast rekonstruiert werden.

Dies ist diagnostisch dahingehend relevant, als die Kontraktilität des rechten/linken Ventrikels in direktem Zusammenhang mit der Entwicklung von Rechts-/Linksherzversagen steht.

Aufgrund der Atmung variiert der Druckgradient zwischen Thorax und Abdomen periodisch, was zu periodischen Schwankungen des Blutstroms in den rechten Ventrikel führt. Diese Änderung der Vorlast führt beim gesunden Herz zu einer Änderung der Kontraktilität (Frank-Starling-Effekt), die an (dP/dt)ₘₐₓ des rechtventrikulären oder pulmonalarteriellen Drucks abgelesen wird. Ein vergleichbarer Effekt tritt auch beim linken Ventrikel auf. Die Atmung führt somit bei beiden Herzkammern und in den herznahen Arterien zu einer Oszillation von (dP/dt)ₘₐₓ.

Der Effekt im rechten Ventrikel (RV) und in der Pulmonalarterie (PA) ist wie folgt:
Beim Einatmen wird im Thorax ein Unterdruck erzeugt, der die thorakalen Gefäße weitet. Hierdurch wird zum einen der Gefäßwiderstand verringert und zum anderen eine Saugwirkung auf das Blut in den angrenzenden Gefäßen ausgeübt. Gleichzeitig wird durch das Absenken des Zwerchfells der Druck im Abdomen erhöht, was dort zu einer Gefäßverengung, somit zu einem Pumpeffekt des Blutes in die angrenzenden Gefäße führt.

Es kommt so während des Einatmens zu einem verstärkten venösen Einstrom von Blut aus dem Abdomen in den Thorax. Die Folge ist eine verstärkte Füllung des rechten Ventrikels, was zu einer Erhöhung der Kontraktiliät führt (Frank-Starling-Effekt). Dies zeigt sich an einer Erhöhung von (dP/dt)ₘₐₓ, das im rechten Ventrikel oder in der Pulmonalarterie gemessen wird.

Die umgekehrte Wirkung ist beim Ausatmen zu beobachten. Somit erhält man eine Oszillation von (dP/dt)ₘₐₓ, welches sein Maximum bei oder kurz nach der maximalen Einatmung erreicht.

Der Effekt im linken Ventrikel (LV) und in der Aorta (Ao) ist wie folgt:
Beim Einatmen werden die Lungen gedehnt, sodass die Kapazität der Lungengefäße zunimmt. Dadurch verringert sich der venöse Rückstrom zum linken Ventrikel. Der verminderten Vorlast entsprechend nimmt die Kontraktilität und damit (dP/dt)ₘₐₓ ab.

Man erhält so eine Oszillation von (dP/dt)ₘₐₓ, welches sein Minimum bei oder kurz nach der maximalen Einatmung erreicht. Der Effekt ist also im LV bzw. der Aorta genau um 180° phasenverschoben zum Effekt im RV bzw. der Pulmonalarterie.

Folgendes sind einige bevorzugte Ausführungsvarianten des erfindungsgemäßen Herzmonitors:
Vorzugsweise umfassen die Mittel zum Bestimmen der Kontraktilität des jeweiligen Ventrikels einen Drucksensor zum Erfassen eines Blutdrucks. Dabei ist eine Positionierung des Sensors im Herzen oder einer herznahen Arterie zweckdienlich.

Auch die Mittel zum Bestimmen der Vorlast umfassen vorzugsweise einen Drucksensor zum Erfassen eines Blutdrucks, der der gleiche Sensor sein kann, wie derjenige, der für das Bestimmen der Kontraktilität durch die Mittel zum Bestimmen der Kontraktilität genutzt wird.

Der Drucksensor kann implantierbar ausgebildet sein und z.B. in eine bei einem implantierbaren Herzstimulator üblicherweise vorgesehene Elektrodenleitung integriert sein.

Anstelle eines Drucksensors oder zusätzlich zu einem Drucksensor zum Erfassen des respiratorischen Verlaufs charakteristischer Punkte des Blutdrucks können auch Mittel zum Erfassen der intrathorakalen Impedanz und/oder des Schlagvolumens vorgesehen sein. Diese können dann ebenfalls sowohl Bestandteil der Mittel zum Bestimmen der Vorlast als auch Bestandteil der Mittel zum Bestimmen der Kontraktilität sein.

Mittel zum Erfassen der intrathorakalen Impedanz und/oder des Schlagvolumens sind grundsätzlich z.B. aus US 7,395,114 bekannt. In Bezug auf die Impedanzmessung werden auch die US 2008/0300504 und die US 2009/0216145 genannt.

In jedem Fall ist ein medizinisches Gerät bevorzugt, das einen Sensor zum Erfassen der Aktivität oder der Lage (z.B. stehend oder liegend) eines Patienten aufweist, also einen Aktivitäts- und/oder Lagesensor. Dieser Sensor ist mit der Auswerteeinheit wenigstens mittelbar verbunden. Als Aktivitätssensor eignet sich beispielsweise ein Blutsauerstoff Sensor, der die Sauerstoffsättigung des Blutes erfasst, oder ein Bewegungssensor.

Vorzugsweise sind die Mittel zum Bestimmen der Vorlast dazu ausgebildet, den thorakalen Druck repräsentierende Druckwerte, insbesondere zu charakteristischen Zeitpunkten des Herzzyklus, z.B. den enddiastolischen oder den maximalen Druck, zu bestimmen und auszuwerten oder zusätzlich oder alternativ eine thorakale Druckdifferenz, d.h. Differenz des thorakalen Drucks zum Minimum des Atemzyklus, zu bestimmen und auszuwerten.

Gemäß einer besonders bevorzugten Ausführungsvariante sind die Mittel zum Bestimmen der Vorlast dazu ausgebildet, beim Bestimmen der Vorlast einen Ausgangswert des Aktivitäts- oder Lagesensors zu berücksichtigen.

Vorzugsweise sind die Mittel zum Bestimmen der Kontraktilität dazu ausgebildet, ein Maß für die Kontraktilität des betrachteten Ventrikels aus Druckwerten, insbesondere den ventrikulären Druck repräsentierenden Druckwerten, abzuleiten. Gemäß einer besonders bevorzugten Ausführungsvariante sind die Mittel zum Bestimmen der Kontraktilität dazu ausgebildet, ein Maß für die Kontraktilität des betrachteten Ventrikels aus dem Maximum der Ableitung des Drucks nach der Zeit, also aus (dP/dt)ₘₐₓ, abzuleiten.

Die Auswerteeinheit ist vorzugsweise dazu ausgebildet, einen funktionalen Zusammenhang zwischen den Kontraktilitätswerten und den Vorlastwerten zu bestimmen. Hierzu ist die Auswerteeinheit vorzugsweise ausgebildet, ein Vorlast-Kontraktilitäts-Diagramm zu erstellen und auszuwerten. Insbesondere kann die Auswerteeinheit dazu ausgebildet sein, Werte eines oder mehrerer der folgenden Parameter aus dem Vorlast-Kontraktilitäts-Diagramm abzuleiten: Minimal- und Maximalwert sowie maximale Steigung. Daneben können Parameter, die aus dem Vorlast-Kontraktilitäts-Diagramm bestimmt wurden, in ihrer Entwicklung über die Zeit betrachtet und Trendparameter bestimmt werden.

Der erfindungsgemäße Herzmonitor umfasst somit ein Sensorsystem zur:
A. Messung einer Größe, aus der sich herzzyklusweise ein Maß für die Vorlast eines Ventrikels bestimmen lässt, vorzugsweise des Blutdrucks (Hierzu wären auch venöse Gefäße geeignet.);
B. Messung einer Größe, aus der sich herzzyklusweise ein Maß für die Kontraktilität des Ventrikels bestimmen lässt, vorzugsweise des Blutdrucks in einem Ventrikel oder einer herznahen Arterie; und
C. optional zur Messung weiterer Größen, z.B. der Aktivität (Sauerstoffsättigung, Bewegungssensoren etc.) oder der Position des Patienten

Die Messgrößen, die gemäß A und B bestimmt werden, können dabei identisch sein.

Die Datenauswertung erfolgt in einem geeigneten System in einem Implantat, einem externen Gerät oder einem Rechenzentrum.

Die Datenauswertung durch die Auswerteeinheit umfasst gemäß einer bevorzugten Ausführungsvariante die folgenden Schritte:
1. Bestimmung eines Maßes für die Vorlast des betrachteten Ventrikels aus der gemäß A bestimmten Messgröße, z.B.
   - thorakaler Druck, insbesondere bestimmt aus charakteristischen Punkten des Herzzyklus, z.B. aus enddiastolischem oder maximalem Druck
   - thorakale Druckdifferenz, d.h. Differenz des thorakalen Drucks zum Minimum des Atemzyklus
      Dabei können auch zusätzliche Sensorgrößen mit einbezogen werden, z.B.
   - Position des Patienten
   - Aktivität des Patienten, z.B. bestimmt aus Bewegungs-/Beschleunigungssensoren, Sauerstoffsättigung etc.
2. Bestimmung eines Maßes für die Kontraktilität des betrachteten Ventrikels aus der gemäß B bestimmten Messgröße, insbesondere (dP/dt)ₘₐₓ
3. Bestimmung des funktionalen Zusammenhangs zwischen den beiden in Schritt 1 und 2 bestimmten Maßen, d.h. Auftragung in einem Vorlast-Kontraktilitäts-Diagramm.
4. Ableitung eines oder mehrerer Parameter aus dem in Schritt 3 bestimmten Diagramm, z.B. Minimal- und Maximalwert, Steigungen etc.

Mit dem beschriebenen System kann der Frank-Starling-Effekt quantifiziert werden, d.h. die Kontraktilität eines der Ventrikel kann vorlastabhängig bestimmt werden. Damit steht eine Möglichkeit zum Erkennen und Überwachen von Herzinsuffizienz des Ventrikels zur Verfügung.

Vorzugsweise wird das oben beschriebene Verfahren parallel im systemischen und im pulmonalen Kreislauf umgesetzt, indem der Herzmonitor entsprechend angepasst ist und z. B. zwei Drucksensoren aufweist, von denen z.B. einer zur Anordnung im rechten Ventrikel oder in der Pulmonalarterie und der andere zur Anordnung im linken Ventrikel oder in der Aorta oder einer peripheren Arterie ausgebildet ist. Die daraus erhaltenen Größen werden verglichen, woraus sich weitere diagnostisch relevante Parameter ableiten lassen.

Die Erfindung soll nun anhand von Ausführungsbeispielen mit Bezug auf die Figuren näher erläutert werden. Von den Figuren zeigt:
- Fig. 1:: ein medizinisches Implantat als Herzmonitor;
- Fig. 2:: ein medizinisches Implantat als Herzmonitor zusammen mit einem externen Gerät;
- Fig. 3:: ein schematisches Blockschaltbild einiger Komponenten des Implantats;
- Fig. 4:: ein schematisches Blockschaltbild einiger Komponenten einer alternativen Ausführungsform des Implantats;
- Fig. 5:: ein schematisches Blockschaltbild einiger Komponenten einer dritten Ausführungsform des Implantats; und
- Fig. 6A and 6B:: die Abhängigkeit von Blutdruck und erster Ableitung des Blutdruckes von der Atmung.

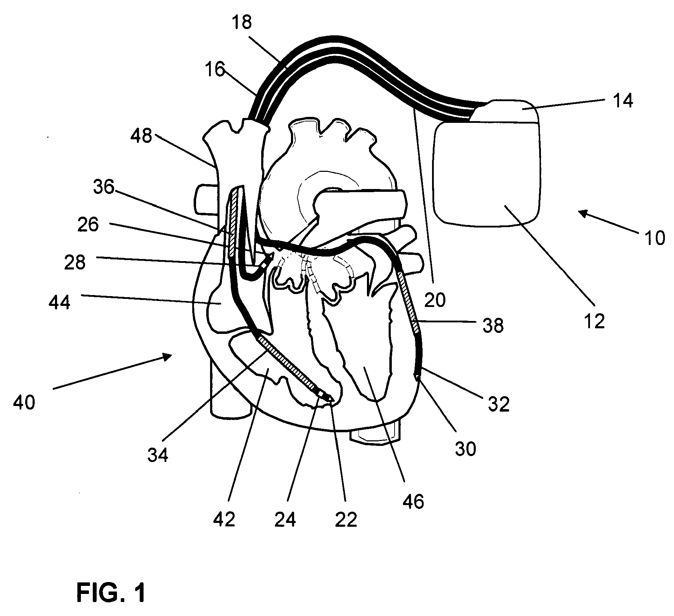

Fig. 1 zeigt einen Herzmonitor in Form eines implantierbaren medizinischen Gerätes, nämlich in Form eines implantierbaren Herzstimulators 10. Der Herzstimulator 10 ist als biventrikulärer Herzschrittmacher und Kardioverter/Defibrillator ausgebildet.

Der Herzstimulator 10 besitzt in an sich bekannter Weise ein Gehäuse 12 aus Metall, das auch als großflächige Elektrode dienen kann. An dem Gehäuse 12 ist ein sog. Header 14 aus Kunststoff angebracht, der als Anschlussgehäuse mit Steckbuchsen entsprechende Stecker von Elektrodenleitungen aufnehmen kann, um auf diese Weise Elektroden an den Elektrodenleitungen elektrisch mit elektrischen Komponenten im Inneren des Gehäuses 12 zu verbinden.

Im abgebildeten Fall sind insgesamt drei Elektrodenleitungen an dem Herzschrittmacher 10 angeschlossen, nämlich eine rechtsventrikuläre Elektrodenleitung 16, eine rechtsatriale Elektrodenleitung 18 und eine linksventrikuläre Elektrodenleitung 20. Jede dieser Elektrodenleitungen trägt an ihrem distalen Ende jeweils ein Paar vergleichsweise kleinflächiger Stimulations- und Sensingelektroden, konkret sind dies eine rechtsventrikuläre Spitzenelektrode RV-TIP 22, eine rechtsventrikuläre Ringelektrode RV-RING 24, eine rechsatriale Spitzenelektrode RA-tip 26 und eine rechtsatriale Ringelektrode RA-tip 28, eine rechtsatriale Elektrodenleitung 18 und schließlich eine linksventrikuläre Spitzenelektrode LV-TIP 30 und eine linksventrikuläre Ringelektrode LV-RING 32 am distalen Ende der linksventrikulären Elektrodenleitung 20.

Zum Zweck einer Defibrillationsschockabgabe sind außerdem noch vergleichsweise großfläche Defibrillationselektroden vorgesehen, die jeweils als Schockwendel ausgebildet sind. Konkret sind dies eine rechtsventrikuläre Schockelektrode RV-COIL 34, welche auf der rechtsventrikulären Elektrodenleitung 16 in der Nähe von deren distalem Ende angeordnet ist. Die rechtsventrikuläre Elektrodenleitung 16 trägt außerdem eine für die Platzierung in der Vena cava vorgesehene proximale Schockelektrode VC-COIL 36. In der Nähe des distalen Endes der linksventrikulären Elektrodenleitung 20 trägt diese eine linksventrikuläre Schockelektrode LV-COIL 38.

Fig. 1 zeigt schematisch, wie die einzelnen Elektroden nach Implantation ungefähr im Herzen angeordnet sind. So zeigt Fig. 1 eine schematische Darstellung eines Herzens 40 mit seinem rechten Ventrikel 42, seinem rechten Atrium 44 und seinem linken Ventrikel 46. Außerdem ist ein Abschnitt der Vena cava superior 48 abgebildet.

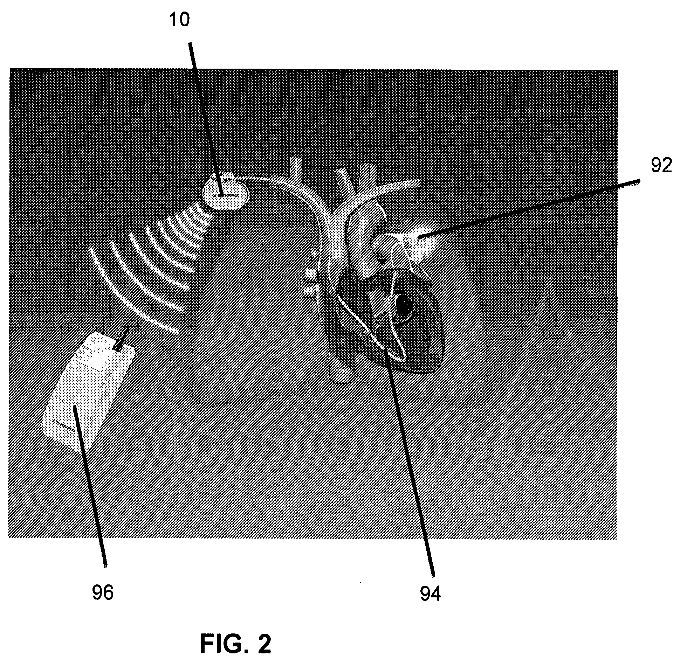

In Figur 2 dargestellt ist ein Drucksensor 92, der über ein Kabel 94 mit dem implantierbaren medizinischen Gerät 10 verbunden ist. Der Drucksensor 92 kann auch einen Temperatursensor enthalten. Der Drucksensor 92 samt Kabel 94 sind so ausgebildet, dass der Drucksensor wie in Figur 2 abgebildet in der Pulmonalarterie positioniert werden kann. Der Drucksensor 92 ist ausgebildet, dem mit der Zeit periodisch schwankenden hydrostatischen Druck in der Pulmonalarterie entsprechende Ausgangssignale in Form von Druckwerten zu liefern.

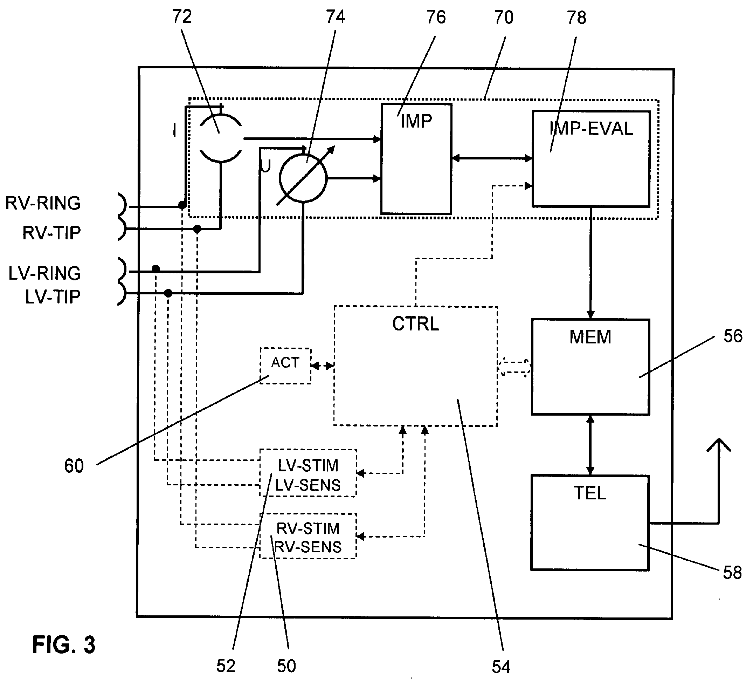

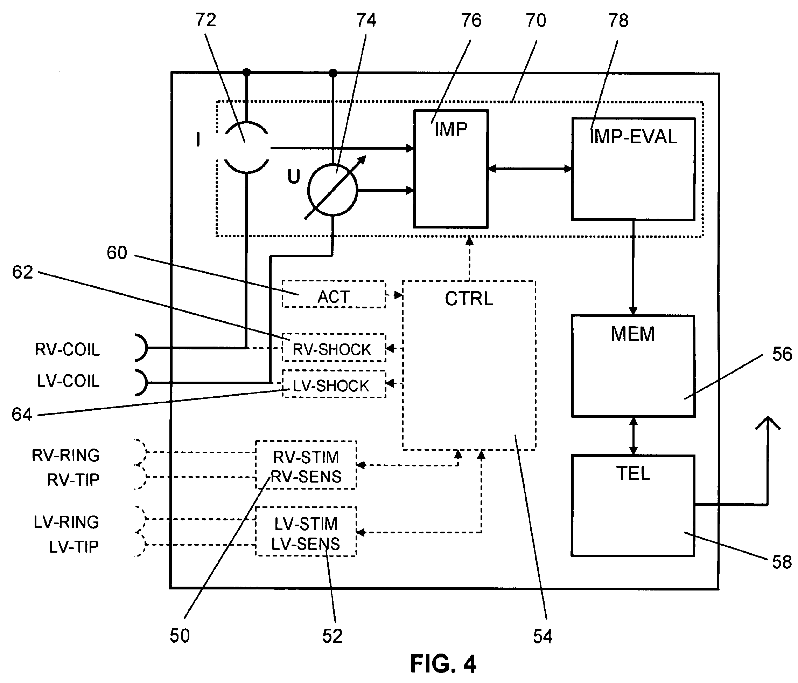

Die Figuren 3 und 4 zeigen einige derjenigen elektrischen bzw. elektronischen Komponenten des Herzstimulators 10, die in dessen Gehäuse 12 angeordnet sind, nämliche eine rechtsventrikuläre Stimulationseinheit RV-STIM und eine rechtsventrikuläre Sensingeinheit RV-SENS, die schematisch dargestellt sind und mit einem gemeinsamen Bezugszeichen 50 versehen sind. Die rechtsventrikuläre Stimulationseinheit und die rechtsventrikuläre Sensingeinheit sind mit dem elektrischen Anschluss für die rechtsventrikuläre Ringelektrode RV-RING und die rechtsventrikuläre Spitzenelektrode RV-TIP verbunden. In gleicher Weise sind eine linksventrikuläre Stimulationseinheit LV-STIM und eine linksventrikuläre Sensingeinheit LV-SENS (gemeinsames Bezugszeichen 52) mit dem elektrischen Anschluss für die linksventrikuläre Spitzenelektrode LV-TIP und die linksventrikuläre Ringelektrode LV-RING verbunden. Die Stimulations- und Sensingeinheiten sind außerdem mit einer zentralen Steuereinheit CTRL 54 verbunden.

Die Steuereinheit ist außerdem mit einem Aktivitätssensor 60 verbunden, der ein Bluttemperatursensor, ein Bewegungssensor oder ein Sensor für die Sauerstoffsättigung des Blutes sein kann und der entsprechend ein Ausgangssignal liefert, das die Aktivität eines Patienten widerspiegelt und daher im Rahmen dieser Beschreibung auch als Aktivitätssignal bezeichnet wird. Die Steuereinheit CTRL 54 ist mit einem Speicher MEM 56 verbunden, der seinerseits wiederum mit einer Telemetrieeinheit TEL 58 verbunden ist. Mittels der Telemetrieeinheit TEL 58 können Daten zu einem externen Gerät 96 (siehe Figur 2) übertragen werden und von diesem Gerät empfangen werden. Das externe Gerät 96 kann hierbei auch als Relaisstation dienen, um Daten aus dem implantierbaren medizinischen Gerät, hier dem implantierbaren medizinischen Herzstimulator 10, zu einem zentralen Servicecenter zu übertragen.

Die Speichereinheit MEM 56 dient dazu, vom Herzstimulator 10 erfasste physiologische oder Betriebsdaten zwischenzuspeichern, wenn diese über die Telemetrie TEL 58 an ein externes Gerät gesendet werden sollen. Außerdem können in der Speichereinheit MEM 56 auch Parameter oder Programmdaten hinterlegt sein, auf die die Steuereinheit CTRL 54 zurückgreift und die den Betrieb des Herzstimulators 10 beeinflussen.

Fig. 4 zeigt außerdem noch, dass für den Fall, dass der Herzstimulator 10 ein Defibrillator ist, auch noch ein rechtsventrikulärer Schockgenerator RV-SCHOCK 62 und ein linksventrikulärer Schockgenerator RV-SCHOCK 64 vorgesehen sein können, die jeweils einerseits mit einem elektrischen Anschluss RV-COIL für die rechtsventrikuläre Schockelektrode bzw. einem Anschluss LV-COIL für die linksventrikuläre Schockelektrode verbunden sein kann sowie andererseits mit der Steuereinheit CTRL 54.

Die Figuren 3 und 4 zeigen in schematischer Darstellung ein Implantat 10 bzw. 10' mit einer Impedanzmesseinheit 70, die eine Stromquelle I 72 und eine Spannungsmesseinheit U 74 sowie Impedanzbestimmungseinheit IMP 76 aufweist.

Die Spannungsmesseinheit U 74 ist bei der Ausführungsvariante in Figur 3 mit einer in einer vom Koronarsinus abzweigenden Lateralvene angeordneten, linksventrikulären Spitzenelektrode LV-TIP sowie einer ebenfalls in einer vom Koronarsinus abzweigenden Lateralvene angeordneten, linksventrikulären Ringelektrode LV-RING verbunden. Die Stromeinspeisungseinheit I 72 ist mit einer rechtsventrikuläre Spitzenelektrode RV-TIP und mit einer rechtsventrikulären Ringelektrode RV-RING - oder genauer gesagt mit Kontakten für den Anschluss dieser Elektroden - verbunden.

In der Ausführungsvariante gemäß Figur 4 ist die Spannungsmesseinheit U 74 zum einen mit der linksventrikulären Defibrillationslektrode LV-COIL und andrerseits mit dem Implantatsgehäuse 12 als zweiter Elektrode verbunden. Die Stromeinspeisungseinheit I 72 ist zum einen mit der rechtsventrikulären Defibrillationslektrode RV-COIL und andrerseits ebenfalls mit dem Implantatsgehäuse 12 als zweiter Elektrode verbunden.

Die Impedanzbestimmungseinheit IMP 76 ist für die Impedanzbestimmung sowohl mit der Stromeinspeisungseinheit I 72 als auch mit der Spannungsmesseinheit U 74 verbunden. Der jeweils ermittelte Impedanzwert wird seitens der Impedanzbestimmungseinheit IMP 76 an eine Auswerteeinheit IMP-EVAL 78 weitergegeben. Die Auswerteeinheit IMP-EVAL 78 bestimmt auf die nachfolgend beschriebene Art und Weise aus den von der Impedanzbestimmungseinheit IMP 76 ermittelten Werten eine enddiastolische Impedanz EDZ und eine endsystolische Impedanz ESZ.

Außerdem leitet die Auswerteeinheit IMP-EVAL 78 aus diesen Werten eine Schlagimpedanz SZ als Differenz aus endsystolischer Impedanz und enddiastolischer Impedanz (SZ = ESZ - EDZ) ab. Dies geschieht in Verbindung mit einem Plausibilitätscheck, währenddessen geprüft wird, ob die enddiastolische Impedanz (EDZ) kleiner ist als die endsystolische Impedanz (ESZ).

Weitere von der Auswerteeinheit IMP-EVAL 78 ermittelte Werte sind für jeden Herzzyklus einen Auswurfanteil (Ejection Fraction = EF), der aus der Schlagimpedanz und der enddiastolischen Impedanz (EF ~ SZ / ESZ, da EF = SV/EDV und SV~SY=SZ/(EDZ*ESZ) sowie EDV∼1/EDZ) oder der enddiastolischen Leitfähigkeit (EDY) und der endsystolischen Leitfähigkeit (ESY) zu bilden ist, sowie eine die Kontraktilität des Herzens repräsentierende Kontraktilitätsgröße. All diese Werte werden von der Auswerteeinheit IMP-EVAL 78 in dem Speicher MEM 56 gespeichert, und zwar vorzugsweise zu regelmäßig wiederkehrenden Speicherzeitpunkten. Einer dieser Parameter oder eine andere aus der intrakardialen (FIG. 3) oder intrathorakalen (FIG. 4) Impedanz bestimmte Größe können alternativ zur Auswertung des Drucksignals als Maß für die schlagweise Kontraktilität bzw. Vorlast herangezogen werden. Neben den weiter oben beschriebenen Elektrodenverschaltungen sind zur Messung der intrakardialen bzw. intrathorakalen Impedanz auch andere Strom- und Spannungspfade denkbar.

Die Auswerteeinheit IMP-EVAL 78 ist weiterhin dazu ausgebildet, für verschiedene A-temphasen jeweils Mittelwerte für die Schlagimpedanz, die EF-Größe oder die Kontraktilität für einen jeweiligen zwischen zwei Speicherzeitpunkten liegenden Zeitraum zu bilden und diese Mittelwerte ebenfalls in dem Speicher zu speichern.

Weiterhin ist die Auswerteeinheit IMP-EVAL 78 ausgebildet, Trends für die von der Auswerteeinheit IMP-EVAL 78 ermittelten Größen zu bestimmen und entsprechende Trendwerte in dem Speicher MEM 56 zu speichern.

Der Speicher MEM 56 ist ausgangsseitig mit der Telemetrieeinheit TEL 58 verbunden, die so ausgebildet ist, dass die in dem Speicher 56 jeweils gespeicherten Werte zu einem regelmäßig wiederkehrenden Sendezeitpunkt von der Telemetrieeinheit 58 mittels einer der Telemetrieeinheit 58 zugeordneten Sendeeinheit derart ausgesandt werden, dass die entsprechenden Werte von einem externen Gerät empfangen und beispielsweise an ein Servicecenter, einen Arzt oder dergleichen weitergeleitet werden können.

Zur Impedanzmessung injiziert die Impedanzmesseinheit 70 einen unterschwelligen Strom zwischen zwei Elektroden der an das Implantat angeschlossenen Elektrodenleitungen und/oder das Implantatsgehäuse 12. Der Strom hat die Form biphasischer Impulse mit konstanter Amplitude. Der durch den Strom hervorgerufene Spannungsabfall (die Spannung) wird über ein anderes Elektrodenpaar der zur Verfügung stehenden Elektroden gemessen. Die gemessene Spannung ist proportional zur Impedanz des Gewebes, welches sich im Messbereich befindet. In einer alternativen Ausführungsvariante können die strominjizierenden Elektroden und die Elektroden zur Spannungsmessung dieselben Elektroden sein.

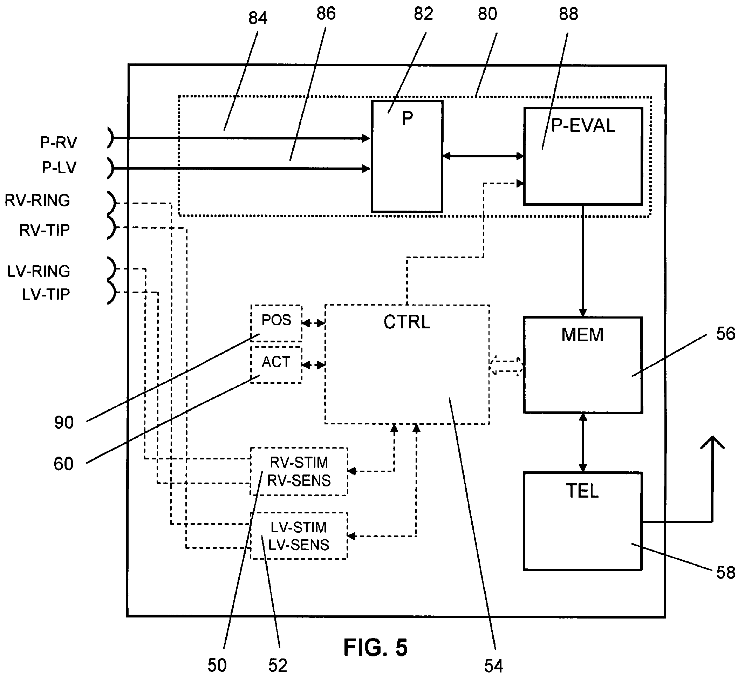

Figur 5 zeigt eine bevorzugte Variante des Herzmonitors, bei der anstelle einer Impedanzmesseinheit eine Blutdruckmesseinheit 80 vorgesehen ist. Die Blutdruckmesseinheit 80 weist eine Druckerfassungseinheit 82 auf, die eingangsseitig einerseits über eine Leitung 84 mit einem Anschluss P-RV für einen rechtsventrikulären Blutdrucksensor verbunden ist sowie über eine Leitung 86 mit einem Anschluss P-LV für einen Blutdrucksensor zur Erfassung des Drucks im linken Ventrikel oder in der Aorta oder in einer peripheren Arterie. Der rechtsventrikuläre Blutdrucksensor ist vorzugsweise im distalen Bereich der rechtsventrikulären Elektrodenleitung 16 angeordnet und liefert Druckwerte des pulmonalen Kreislaufs, während ein linksventrikulärer Drucksensor entsprechend Druckwerte aus dem systemischen Kreislauf liefert. Die von den Drucksensoren gelieferten Druckwerte werden durch die Druckerfassungseinheit 82 aufbereitet. Mit der Druckerfassungseinheit 82 verbunden ist eine Auswerteeinheit P-EVAL 88, die die aufbereiteten Druckwerte wie nachfolgend beschrieben auswertet.

Das Erfassen und Auswerten der Druckwerte läuft dabei wie folgt ab:
Mit dem von der Blutdruckmesseinheit 80 gebildeten Sensorsystem erfolgt eine Messung des Blutdrucks im rechten Ventrikel und in der pulmonalen Arterie. Optional erfolgt über einen Positionssensor 90 eine Erfassung der Position des Patienten, um Liegen und Stehen getrennt zu behandeln.

Die so erfassten Werte werden von der Auswerteeinheit 88 wie folgt ausgewertet:
1. Bestimmen der Atemdruckkurve aus charakteristischen Punkten des Blutdrucksignals, z.B. aus dem Maximum oder dem enddiastolischen Druck jedes Herzzyklus'
2. Bestimmen von (dP/dt)ₘₐₓ pro Herzzyklus
3. Auftragen der Punkte [Atemdruck; (dP/dt)ₘₐₓ] jedes Zyklus' in einem Diagramm
4. Bestimmen geeigneter Parameter aus diesem Diagramm, z.B.
   - Minimal-/Maximalwerte, Perzentile
   - Streuungsmaße (Eine krankhafte Veränderung der Kontraktilität könnte sich auch in einer verstärkten Streuung bei gleicher Vorlast zeigen.)
   - Steigungsparameter (z. B. mittlere Steigung, maximale Steigung, Steigung relativ zu einem aus dem Diagramm abgeleiteten Wert) oder Parameter geeigneter Fitfunktionen (evtl. auch Test unterschiedlicher Fitfunktionen, um eine krankhaft bedingte Änderung der *Form* der Abhängigkeit Kontraktilität(Vorlast) zu erkennen)

Der in Schritt 4 bestimmte Parameter kann mit geeigneten Referenzwerten, z.B. früheren Messungen oder vorgegebenen Schwellwerten verglichen werden, die im Speicher MEM 56 gespeichert sein können. Aus dem zeitlichen Verlauf des Parameters oder der Differenz zum Referenzwert lassen sich weitere Parameter ableiten, insbesondere Trendparameter.

Die Anzeige kann in geeigneten externen Geräten erfolgen, z.B. im Rahmen von Home Monitoring oder einem eigenständigen Gerät.

Vorzugsweise ist die Auswerteeinheit oder eine separate Analyseeinheit dazu ausgebildet, einen Schwellvergleich derart durchzuführen, dass bei Über-/Unterschreiten eines Schwellwerts unterschiedliche Aktionen ausgelöst werden können, z.B.
- Alarmsignal an den behandelnden Arzt
- Alarmsignal/Anweisungen an den Patienten, z.B. Aufforderung zur Einnahme von Medikamenten oder einen Arzt aufzusuchen/zu kontaktieren
- Steuerung eines Therapiegeräts, z.B. Anpassung von Pacing-Parametern eines Schrittmachers/ICDs, Abgabe einer antitachykarden Therapie (z.B. Defibrillationsschock)
- Medikamentengabe

Anstelle des respiratorischen Verlaufs charakteristischer Punkte des Blutdrucks als Sensorgröße A sind auch andere Größen denkbar, z.B.:
- intrathorakale Impedanz

Anstelle von (dP/dt)ₘₐₓ als Sensorgröße B sind gemäß der Ausführungsvarianten, die in Figuren 2 und 3 abgebildet sind, auch denkbar:
- intrakardiale Impedanz
- Schlagvolumen

Der Herzmonitor ist im in Figur 5 dargestellten Ausführungsbeispiel dazu ausgebildet, Blutdruckmessungen sowohl im systemischen als auch im pulmonalen Kreislauf durchzuführen, z.B. durch Blutdruckmessung in beiden Ventrikeln. Hierdurch ist auch die Erkennung von obstruktiver Apnoe möglich, welche durch intrathorakale Impedanzmessung allein nicht gut detektierbar ist.

Wenn bei einem Apnoeereignis die Luftröhre verschlossen wird, kann sich die Lunge nicht dehnen. Aus diesem Grund erfolgt keine Verminderung der Vorlast des linken Ventrikels, wie sie oben beschrieben wurde, sodass auch keine Variation in (dLVP/dt)ₘₐₓ bzw. (dA-oP/dt)ₘₐₓ beobachtet wird. LVP ist hier der Druck im linken Ventrikel bzw. der Ausgangswert eines zum Platzieren im linken Ventrikel vorgesehen Drucksensors. AoP ist hier der Druck in der Aorta bzw. der Ausgangswert eines zum Platzieren in der Aorta vorgesehenen Drucksensors.

Der thorakale Druck wird dagegen ganz regulär abgesenkt, sodass (dRVP/dt)ₘₐₓ bzw. (dPAP/dt)ₘₐₓ die üblichen Schwankungen aufweisen. RVP ist hier der Druck im rechten Ventrikel bzw. der Ausgangswert eines zum Platzieren im rechten Ventrikel vorgesehenen Drucksensors. PAP ist hier der Druck in der Pulmonalarterie bzw. der Ausgangswert eines zum Platzieren in der Aorta vorgesehenen Drucksensors.

Eine Verminderung der (dP/dt)ₘₐₓ-Amplitude im systemischen Kreislauf bei gleich bleibender Amplitude im pulmonalen Kreislauf ist daher ein Hinweis auf ein Event obstruktiver Schlafapnoe. Die Auswerteeinheit ist gemäß einer bevorzugten Ausführungsvariante dazu ausgebildet, eine derartige Verminderung der (dP/dt)ₘₐₓ-Amplitude im systemischen Kreislauf bei gleich bleibender Amplitude im pulmonalen Kreislauf zu detektieren und ein Apnoewarnsignal zu generieren. Eine alternative Ausführung sieht vor, bei Detektion des Beginns oder Endes eines Apnoeereignisses eine geeignete Therapie zu starten, zu modifizieren oder zu beenden. Eine weitere alternative Ausführung sieht vor, Apnoeereignisse lediglich zu überwachen und die ermittelten Daten, z.B. Anzahl, Zeitpunkte des Auftretens, Dauer, Stärke des gemessenen Effekts, statistische Kenngrößen dieser Daten etc., in geeigneter Weise darzustellen, beispielsweise in einem externen Gerät oder über Home Monitoring.

Außerdem ist die Auswerteeinheit gemäß einer weiteren bevorzugten Ausführungsvariante dazu ausgebildet, eine Unterscheidung von aktiver und passiver Atmung durchzuführen.

Beim selbsttätigen Einatmen erzeugt der Patient im Thorax einen Unterdruck, durch den Luft in die Lungen gesaugt wird; dagegen wird bei künstlicher Beatmung Luft durch einen Überdruck in die Lungen gepresst. Daraus folgt, dass aktive und passive Atmung zwar denselben Verlauf des Lungenvolumens besitzen, die thorakalen Drücke aber gegenphasig sind. Wie oben beschrieben wurde, wirkt sich das Lungenvolumen über den Frank-Starling-Mechanismus auf die linksventrikuläre Kontraktilität aus, während der Thoraxdruck sich in einer Amplitudenschwankung der charakteristischen Punkte jedes Herzzyklus niederschlägt (besonders deutlich im pulmonalen Kreislauf). Damit gibt die Phasenbeziehung zwischen (dLVP/dt)ₘₐₓ bzw. (dAoP/dt)ₘₐₓ und Pₜₕₒᵣₐₖₐₗ (z.B. aus PAP) Hinweise darauf, ob der Patient selbsttätig atmet oder beatmet wird.

Die Auswerteeinheit ist vorzugsweise dazu ausgebildet, die Phasenbeziehung zwischen (dLVP/dt)ₘₐₓ bzw. (dAoP/dt)ₘₐₓ und Pₜₕₒᵣₐₖₐₗ zu erfassen.

Der erfindungsgemäße Herzmonitor erlaubt es, den Frank-Starling-Mechanismus zu erfassen, d.h. die Abhängigkeit der Kontraktilität von der Vorlast. Damit wird ein wesentlich detaillierteres Bild der Leistungsfähigkeit des untersuchten Ventrikels erhalten als durch eine bloße Mittelung aller (dP/dt)ₘₐₓ-Werte.

Die Variation der Vorlast erfolgt spontan, muss also nicht von außen, z.B. durch ein Messprotokoll, vorgegeben werden.

Wie ausgeführt, ist anstelle einer druckbasierten Implementierung des Herzmonitors auch eine impedanzbasierte Umsetzung denkbar, die auf die gängigen Sensorsysteme von Schrittmachern/ICDs zurückgreifen kann.

Die Datenauswertung kann anstelle durch eine Auswerteeinheit eines implantierbaren medizinischen Gerätes auch in einem externen Gerät, z.B. einem Patientengerät 96 (siehe Figur 2) oder einem zentralen Servicecenter erfolgen. Hierzu können entsprechende Blutdruckwerte oder Impedanzwerte mittels einer Telemetrieeinheit an das jeweilige externe Gerät übertragen werden.

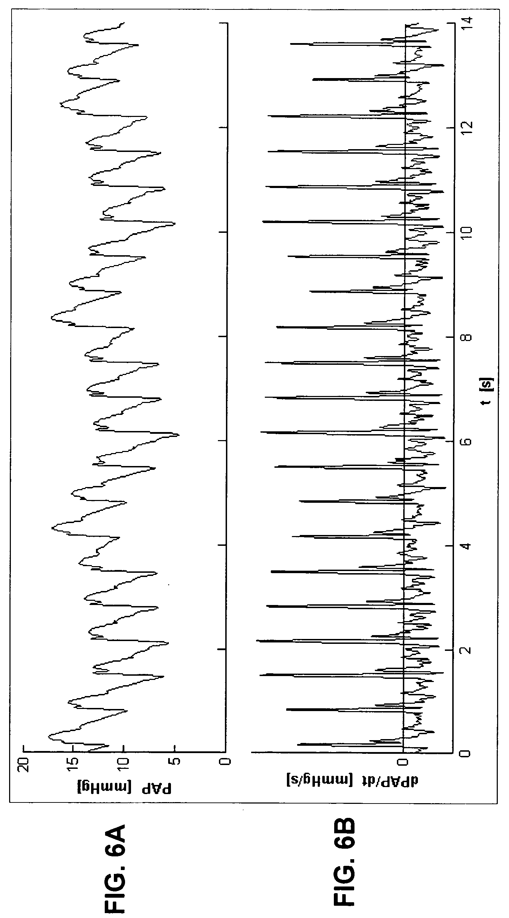

In Figur 6A ist der Blutdruck in der Pulmonalarterie über 14 Sekunden bzw. über drei A-temzyklen bzw. 21 Herzaktionen hinweg aufgetragen. Die atmungsbedingten Schwankungen im Thoraxdruck zeigen sich an Oszillationen des enddiastolischen Drucks (Minimum eines jeden Herzzyklus') sowie im Maximaldruck.

In Figur 6B ist die Ableitung des Blutdrucks nach der Zeit gezeigt. Die Peaks nach oben entsprechen (dP/dt)max in jedem Herzzyklus. Die Amplituden oszillieren mit ca. 180° Phasenverschiebung zum Thoraxdruck.
Die Blutdruckmessung erfolgte im Tierversuch am Schaf unter Beatmung.

## Patentansprüche

1. Medizinisches Gerät mit zwei Drucksensoren (P-RV, P-LV), von denen der erste Drucksensor (P-RV) ausgebildet ist, den Druck im rechten Ventrikel eines Herzens zu erfassen und ein entsprechendes erstes Drucksignal abzugeben und der zweite Drucksensor (P-LV) ausgebildet ist, den Druck im linken Ventrikel des Herzens zu erfassen und ein entsprechendes zweites Drucksignal abzugeben,
Mitteln zum Bestimmen der Vorlast des rechten Ventrikels für einen jeweiligen Herzzyklus als Maximum des ersten Drucksignals,
Mitteln zum Bestimmen der Vorlast des linken Ventrikels für einen jeweiligen Herzzyklus als Maximum des zweiten Drucksignals,
Mitteln zum Bestimmen der Kontraktilität des rechten Ventrikels für einen jeweiligen Herzzyklus aus dem Maximum der Ableitung des ersten Drucksignals nach der Zeit,
Mitteln zum Bestimmen der Kontraktilität des linken Ventrikels für einen jeweiligen Herzzyklus aus dem Maximum der Ableitung des zweiten Drucksignals nach der Zeit, und
eine Auswerteeinheit (88), die mit den Mitteln zum Bestimmen der Vorlast und den Mitteln zum Bestimmen der Kontraktilität verbunden und dazu ausgebildet ist, für jeden Ventrikel einen funktionalen Zusammenhang zwischen den Kontraktilitätswerten und den Vorlastwerten zu bestimmen.

2. Medizinisches Gerät nach Anspruch 1, **dadurch gekennzeichnet, dass** das medizinische Gerät einen Sensor zum Erfassen der Aktivität oder der Lage eines Patienten aufweist.

3. Medizinisches Gerät nach einem der Ansprüche 1 bis 2, **dadurch gekennzeichnet, dass** die Mittel zum Bestimmen der Vorlast dazu ausgebildet sind, eine thorakale Druckdifferenz zu bestimmen und auszuwerten.

4. Medizinisches Gerät nach einem der Ansprüche 1 bis 3, **dadurch gekennzeichnet, dass** die Mittel zum Bestimmen der Vorlast dazu ausgebildet sind, beim Bestimmen der Vorlast einen Ausgangswert des Sensors zum Erfassen der Aktivität oder der Lage eines Patienten zu berücksichtigen.

5. Medizinisches Gerät nach einem der Ansprüche 1 bis 4, **dadurch gekennzeichnet, dass** die Auswerteeinheit dazu ausgebildet ist, für jeden Ventrikel ein Vorlast-Kontraktilitäts-Diagramm zu erstellen und auszuwerten.

6. Medizinisches Gerät nach Anspruch 5, **dadurch gekennzeichnet, dass** die Auswerteeinheit dazu ausgebildet ist, Werte eines oder mehrerer der folgenden Parameter aus dem Vorlast-Kontraktilitäts-Diagramm abzuleiten: Minimal- und Maximalwert, mittlere/maximale Steigung und Steigung an ausgezeichneten Punkten sowie Parameter aus Kurvenfits.

7. Medizinisches Gerät nach einem der Ansprüche 1 bis 6, **dadurch gekennzeichnet, dass** die Auswerteeinheit ausgebildet ist, eine Verminderung des Maximums der Ableitung des zweiten Drucksignals nach der Zeit bei gleich bleibenden Maximum der Ableitung des ersten Drucksignals nach der Zeit zu detektieren und dann ein Apnoewarnsignal zu generieren.

## Claims

1. A medical device comprising two pressure sensors (P-RV, P-LV), of which the first pressure sensor (P-RV) is configured to detect the pressure in the right ventricle of a heart and to output a corresponding first pressure signal and the second pressure sensor (P-LV) is configured to detect the pressure in the left ventricle of the heart and to output a corresponding second pressure signal,
means for determining the preload of the right ventricle for a particular cardiac cycle as maximum of the first pressure signal
means for determining the preload of the left ventricle for a particular cardiac cycle as maximum of the second pressure signal,
means for determining the contractility of the right ventricle for a particular cardiac cycle from the maximum of the derivative of the first pressure signal according to time,
means for determining the contractility of the left ventricle for a particular cardiac cycle from the maximum of the derivative of the second pressure signal according to time, and
an evaluation unit (88), which is connected to the means for determining the preload and the means for determining the contractility and which is configured to determine, for each ventricle, a functional relationship between the contractility values and the preload values.

2. The medical device according to claim 1, **characterised in that** the medical device has a sensor for detecting patient activity or patient position.

3. The medical device according to either one of claims 1 or 2, **characterised in that** the means for determining the preload are configured to determine and to evaluate a thoracic pressure differential.

4. The medical device according to any one of claims 1 to 3, **characterised in that** the means for determining the preload are configured, when determining the preload, to take into consideration a starting value of the sensor for detecting the activity or the position of a patient.

5. The medical device according to any one of claims 1 to 4, **characterised in that** the evaluation unit is configured to create and to evaluate a preload-contractility graph for each ventricle.

6. The medical device according to claim 5, **characterised in that** the evaluation unit is configured to derive values of one or more of the following parameters from the preload-contractility graph: minimum and maximum value, average/maximum gradient and gradient at marked points, and also parameters from curve fits.

7. The medical device according to any one of claims 1 to 6, **characterised in that** the evaluation unit is configured to detect a reduction of the maximum of the derivative of the second pressure signal according to time with constant maximum of the derivative of the first pressure signal according to time and to then generate an apnea warning signal.

## Revendications

1. Appareil médical avec deux capteurs de pression (P-RV, P-LV), parmi lesquels le premier capteur de pression (P-RV) est conçu pour déterminer la pression dans le ventricule droit d'un coeur et délivrer un premier signal de pression correspondant, et le second capteur de pression (P-LV) est conçu pour déterminer la pression dans le ventricule gauche du coeur et délivrer un second signal de pression,
des moyens pour la détermination de la précharge du ventricule droit pour un cycle cardiaque respectif en tant que maximum du premier signal de pression,
des moyens pour la détermination de la précharge du ventricule gauche pour un cycle cardiaque respectif en tant que maximum d'un deuxième signal de pression,
des moyens pour la détermination de la contractilité du ventricule droit pour un cycle cardiaque respectif à partir du maximum de la dérivée du premier signal de pression par rapport au, temps,
des moyens pour la détermination de la contractilité du ventricule gauche pour un cycle cardiaque respectif à partir du maximum de la dérivée du deuxième signal de par rapport au, temps, et
une unité de traitement (88) qui avec les moyens pour la détermination de la précharge et les moyens pour la détermination de la contractilité sont reliés et conçus pour déterminer pour chaque ventricule, une correspondance fonctionnelle entre les valeurs de contractilité et les valeurs des précharges.

2. Appareil médical selon la revendication 1, **caractérisé en ce que** l'appareil médical présente un capteur pour la détection de l'activité ou la position d'un patient.

3. Appareil médical selon l'une des revendications 1 à 2, **caractérisé en ce que** les moyens pour la détermination de la précharge sont conçus pour déterminer et évaluer une différence de pression thoracique.

4. Appareil médical selon l'une des revendications 1 à 3, **caractérisé en ce que** les moyens pour la détermination de la précharge sont conçus pour, lors de la détermination de la précharge, tenir compte de l'activité ou de la position d'un patient pour la détection d'une valeur de sortie du capteur.

5. Appareil médical selon l'une des revendications 1 à 4, **caractérisé en ce que** l'unité de traitement est conçue pour établir et exploiter un diagramme de contractilité de précharge pour chaque ventricule.

6. Appareil médical selon la revendication 5, **caractérisé en ce que** l'unité de traitement est conçue pour dériver un ou plusieurs des paramètres suivants à partir du diagramme de contractilité de précharge : valeurs minimale et maximale, pente moyenne/maximale et pente à des points particuliers désignés ainsi que des paramètres à partir de courbes types.

7. Appareil médical selon l'une des revendications 1 à 6, **caractérisé en ce que** l'unité de traitement est conçue pour détecter une diminution du maximum de la dérivée du second signal de pression après le temps pour un maximum de la dérivée du premier signal de pression restant constant après le temps et d'ensuite générer un signal d'avertissement d'apnée.
